# EUROPEAN PATENT APPLICATION

(11) **EP 0 533 263 A2**
(43) Date of publication of application: **24.03.1993**
(21) Application number: 92202791.7
(22) Date of filing: 12.09.1992
(51) Int. Cl.: C12N 15/51, C12N 7/04, A61K 39/29

(54) **A multivalent hepatitis B virus vaccine**

(30) Priority: 20.09.1991 US 762676
(71) Applicant: MERCK & CO. INC., Rahway New Jersey 07065-0900 (US)
(72) Inventor: Kniskern, Peter J., Lansdale, PA 19446 (US); Hagopian, Arpi, Lansdale, PA 19446 (US); Burke, Pamela, Lansdale, PA 19446 (US)
(74) Representative: Barrett-Major, Julie Diane

(57) **Abstract**

In order to produce a mixture of different serotypes of hepatitis B virus (HBV) surface proteins in the form of particles, DNA encoding two or more HBV proteins were expressed by a single recombinant yeast host. These HBV proteins display the antigenic sites genetically encoded by the S domain (including the preS domain) of the HBV virion envelope open reading frame and when expressed in a yeast host deficient in its ability to glycosylate, contain substantially reduced levels of entrapped carbohydrate when compared with HBsAg particles produced in yeast cells which are "wild-type" for glycosylation. These particles are useful as a vaccine for both the active and passive treatment or prevention of disease and/or infection caused by HBV or other agents serologically related to HBV including antigenic variants in the immunodominant epitopes of the surface protein, and are also useful as antigens and immunogens for development of diagnostic tests for such diseases or infections.

## Description

### BACKGROUND OF THE INVENTION

Hepatitis B virus (HBV) is the infectious agent responsible for several varieties of human liver disease. Many individuals who are infected by HBV suffer through an acute phase of disease, which is followed by recovery. However, a percentage of infected individuals fail to clear their infection, thereby becoming chronic carriers of the infection. HBV infection is endemic in many parts of the world, with a high incidence of infection occurring perinatally from chronically infected mothers to their newborns who themselves often remain chronically infected. The number worldwide has been estimated at over three hundred million. From this pool of carriers, hundreds of thousands die annually from the long-term consequences of chronic hepatitis B (cirrhosis and/or hepatocellular carcinoma).

The hepatitis B delta virus is an agent which, during coinfection with HBV, is responsible for an acute fulminating disease with a generally fatal resolution. The delta virus does not encode (from its own genetic material) proteins which serve as the virion envelope; rather, the delta virus genome is encapsidated within the envelope proteins encoded by the coinfecting HBV, thereby sharing a close structural and immunologic relationship with the HBV proteins which are described below. It is unknown at this time whether other infectious agents share similar relationships with HBV. However, it is clear that proteins with expanded breadth of serologic re- activity or enhanced immunogenic potency would be useful in systems for diagnosis or prevention (or treatment) of diseases (or infections) by a class of agents with even slight or partial antigenic cross-reactivity with HBV.

The HB virion is composed of two groups of structural proteins, the core proteins and the envelope or surface proteins. In addition to being the major surface proteins of the virion, i.e., Dane particle, the envelope proteins also are the major constituents of Australia antigen, or 22 nm particles. These envelope proteins are the translational products of the large viral open reading frame (ORF) encoding at least 389 amino acids (aa). This ORF is demarcated into three domains, each of which begins with an ATG codon that is capable of functioning as a translational initiation site in vivo. These domains are referred to as preS1 (108 aa), preS2 (55 aa), and S (226 aa) in their respective 5'-3' order in the gene. Thus, these domains define three polypeptides referred to as S or HBsAg (226 aa), preS2+S (281 aa), and preS1+preS2+S (389 aa), also referred to as p24/gp27, p30/gp33/gp36 and p39/gp42 respectively (as well as the major, middle and large proteins).

The envelope proteins of HBV are glycoproteins with carbohydrate side chains (glycans) attached by N-glycosidic linkages to defined peptide recognition sites. [Heermann et al., J. Virol. 52, 396 (1984) and Stibbe et al., J. Virol. 46, 626 (1983)]. Thus, the HBV polypeptides produced during natural infection comprise the species p24/gp27 (the S polypeptide and its glycosylated derivative), gp33/gp36 (the preS2+S polypeptide glycosylated in the preS2 domain only and the same polypeptide glycosylated in the S as well as the preS2 domain), and p39/gp42 (the preS1+preS2+S peptide and its derivative glycosylated in the preS1 domain). Currently available plasma-derived vaccines are composed of proteins containing virtually only the S domain (comprising the p24 monomer and its glycosylated derivative gp27), while yeast-derived vaccines successfully developed to date are composed exclusively of the S polypeptide (comprising exclusively the nonglycosylated p24 species).

The 22 nm HBsAg particles, have been purified from the plasma of chronic carriers. In terms of their plasma being particle-positive, these chronic carriers are referred to as HBs⁺. If infected persons have mounted a sufficient immune response, they can clear the infection and become HBs⁻. In terms of their formation of antibodies to HBs, these individuals are denoted anti-HBs⁺. In this way, anti HBs⁺ is correlated with recovery from disease and with immunity to reinfection from disease and with immunity to reinfection with HBV. Therefore, the stimulation or formation of anti-HBs by HB vaccines has been expected to confer protection against HBV infection.

This hypothesis has been testable experimentally. Outside of man, the chimpanzee is one of the few species which is fully susceptible to HBV infection , as reflected in quantifiable markers such as HBs⁺ and elevated serum levels of liver enzymes. Chimpanzees have been vaccinated with three doses of purified HBsAg particles and then challenged intravenously with infectious HBV. While mock-vaccinated animals have shown signs of acute HBV infection, the HBsAg-vaccinated animals have been protected completely from signs of infection. Therefore, in this experimental system, HBsAg particles, composed of p24 (or p24 and gp27), have been sufficient to induce protective immunity. Spurred by these observations, several manufacturers have produced HB vaccines composed of HBsAg particles.

Recently, several independent lines of evidence have suggested that the preS sequences may be important in conferring immunity to HBV. The immune elimination of preS antigens during the course of viral infection appears prognostic for viral clearance and abrogation of infection [Budkowska et al., Ann. Inst. Past./Immun., 136D:56-65, (1985)]. During acute hepatitis B infection, antibodies to the preS domains often arise earlier than antibodies to S [Petit et al., Mol. Immun., 23:511-523, (1986)]. In inbred mice, the immune responses to S and preS appear to be regulated independently, and the presence of the preS domain can influence the immune response to S [Milich et al., Proc. Nat. Acad. Sci. USA, 82:8168-8172, (1985), J. Immunol., 137:315-322 (1986); Neurath et al., J. Med. Virol., 17:119-125, (1985)]. Furthermore, antibodies to the preS domain neutralize viral infectivity in vitro [Neurath et al., Vaccine, 4:35-37, (1986)], and preS antigens protect immunized chimpanzees against HBV infection [Itoh et al., Proc. Nat. Acad. Sci. USA, 83:9174-9178, (1986)]. In light of these observations and because, as discussed below, of the utility of recombinant yeast in producing HB vaccines from recombinant Saccharomyces cerevisiae, we have formulated experimental HB vaccines from recombinant S. cerevisiae.

In order to expand the available supply of HB vaccines, manufacturers have turned to recombinant DNA technology to mediate the expression of viral envelope proteins. Among microbial systems, Escherichia coli and S. cerevisiae have been used most commonly for the expression of many recombinant-derived proteins. Numerous attempts to express immunologically active HBsAg particles in E. coli have been unsuccessful. However, S. cerevisiae has shown great versatility in its ability to express immunologically active HBsAg particles. These particles (composed exclusively of p24), when formulated into a vaccine, have proven capable of fully protecting chimpanzees against challenge with live HBV of diverse serotypes. Furthermore, yeast-derived S particles are also immunologically active and as effective in preventing disease or infection in human clinical trials as plasma-derived HBsAg [Stevens et al., JAMA, 257:2612-2616 (1987)]. Therefore, the utility of S. cerevisiae as a host species for directing the synthesis of recombinant HBsAg is established firmly. In addition, expression of human therapeutic agents and vaccines in yeast can be very useful for product development, since yeast is free of endotoxin, is nonpathogenic to man, can be fermented to industrial scale, and lacks many of the safety concerns which surround the use of continuous mammalian cell lines (many of which are virally transformed, may be tumorigenic in mice and all of which contain protooncogenes).

S. cerevisiae (bakers' yeast) is a eukaryote which is capable of synthesizing glycoproteins. Protein glycosylation in yeast has been the subject of numerous recent review articles [notably: Kukuruzinska et al., Ann. Rev. Biochem., (1987) 56, 915-44; Tannen et al., BBA, (1987) 906, 81-99].

As expected by their traversal of the rough endoplasmic reticulum and Golgi apparatus, foreign proteins can undergo both N- and 0-linked glycosylation events. This glycosylation or addition of glycans to appropriate receptor amino acids (aa) on the polypeptide occurs either at specific serine (Ser) or threonine (Thr) residues (O-linked) or at specified asparagine (Asn) residues (N-linked). The specificity for O-linked addition at Ser or Thr residues is not clearly understood and is determined empirically on a case-by-case basis.

The signal sequence for N-linked glycosylation is well defined as either of the amino acid sequences Asn-X-Thr or Asn-X-Ser (wherein X is any amino acid). In addition to synthesizing many autologous, native, glycosylated proteins (among them being those called mannoproteins, or mannopeptides), yeast also are capable of glycosylating heterologous or foreign proteins expressed by recombinant technology (if the heterologous protein contains the appropriate glycosylation signal sequence for either N-linked or O-linked glycosylation).

The preS2+S polypeptides, which are produced during natural infection contain no more than two "core" [ca. 3 kilodaltons (kD) in size] N-linked glycans, one in the S region and a second on the Asn at amino acid residue 4 of the preS2 domain. The recognition site in the S domain is not glycosylated in either Recombivax HB® or in recombinant preS2+S synthesized in yeast. However, the site at amino acid residue 4 of the preS2 domain is recognized and glycosylated by yeast.

The preS1 domain contains an N-linked glycosylation site at amino acid residue 4 of the preS1 region and a potential site at aa residue 26 for serotype adw. It is readily apparent to those skilled in the art that arguments set forth for preS2 glycosylation also will follow for diverse sequences in the preS2 region as well as for those in the preS1 and S domains.

Yeast synthesizing recombinant preS1+preS2+S or preS2+S add a "core" glycan which is similar to that added to the native polypeptide during viral infection. However, if the yeast host cell is "wild-type" for glycosylation (i.e., containing the full complement of enzymes required for native glycosylation which is the case for virtually all commonly used yeast strains), a significant number of these glycans are extended with a large number of additional mannose residues in a manner identical to that employed by yeast in making its own structural mannoproteins. This extended addition of the glycan, when it occurs on a foreign gene product such as the preS2+S polypeptide, is referred to as hyperglycosylation. It is readily apparent to those skilled in the art that arguments set forth for yeast also will extend to other host cells (e.g., insect, fungi, etc.) which may be subject to divergent glycosylation patterns.

Furthermore, it has been demonstrated that recombinant forms of 22nm particles of HBV surface proteins expressed in wild-type yeast host cells, may entrap substantial amounts of yeast cell carbohydrate (deriving at least in part from the structural mannoproteins and mannopeptides of the yeast host cell) within the 22nm particle. This entrapped carbohydrate could pose potential problems in that the entrapped carbohydrate may cause the generation of antibodies against yeast carbohydrate moieties on glycosylated proteins, and a vaccine immunogen containing entrapped yeast carbohydrate would react with anti-yeast antibodies present in most mammalian species thereby potentially diminishing its effectiveness as an immunogen and vaccine.

Hyperglycosylation and entrappment of complete mannoproteins and mannopeptides may be eliminated or glycosylation limited in HBV preS and S containing polypeptides and their corresponding particles by any of the following approaches.

Firstly, N-linked hyperglycosylation may be prevented or limited during growth of the recombinant host through the presence in the growth medium of an exogenous agent (e.g., tunicamycin). Secondly, polypeptides, from recombinant or natural sources may be deglycosylated either chemically (e.g. anhydrous trifluoromethane- sulfonic acid or anhydrous hydrogen fluoride) or enzymatically (e.g., with N-glycanase, Endo-F or Endo-H) or physically (e.g. sonication). Thirdly, the recognition site for glycosylation may be changed or deleted by mutagenesis at the DNA level, such that core glycosylation, and thereby hyperglycosylation as well, is prevented. Such modified preS+S ORE's in which the glycosylation recognition sequence has been altered (directed by suitable promoters active in yeast) have been transformed into yeast host cells. The resultant preS+S polypeptides lack glycosylation. Fourthly, host cells may be identified which lack critical enzymes required for glycosylation, which illustrates the present invention without however limiting the same thereto. One such yeast strain has been identified (mnn9- mutant) [Ballou, L et al., (1980), J.Biol.Chem., 255, pp 5986-5991] which lacks a critical enzyme in the glycosylation pathway necessary for the elongation (hyperglycosylation) of the N-linked glycans; chemical studies indicate that this mutant makes mannoproteins without outer-chain mannose residues and containing only the "core" carbohydrate [Ballou, C.E. et al., (1986), Proc.Natl.Acad.Sci.U.S.A., 83, pp 3081-3085; Tsai, P. et al., (1984), J.Biol.Chem., 259, pp 3805-3811]. The ORF for the S or preS+S polypeptide (transcription directed by suitable promoters active in yeast) has been used to transform such mnn9⁻ mutant yeast. The resulting preS+S polypeptide (where preS can S1, or preS2, or preS1+preS2) contains only "core" glycosylation and lacks hyperglycosylation.

Although the S polypeptides are neither glycosylated nor hyperglycosylated when expressed in yeast, particles composed therefrom contain significant levels of entrapped carbohydrate deriving from yeast mannopeptide. Therefore, the expression of S polypeptides as well as preS containing polypeptides in yeast cells which cannot hyperglycosylate results in decreased levels of carbohydrate in the expressed 22nm particles.

S. cerevisiae has shown great versatility in its ability to express immunologically active 22 nm particles. These particles, when formulated into a vaccine, have proven capable of fully protecting chimpanzees against challenge with live HBV. Furthermore, yeast-derived HBsAg has been effective immunologically in human clinical trials as plasma-derived HBsAg. Therefore, the utility of S. cerevisiae as a host species for directing synthesis of recombinant HBsAg is established firmly.

In a variety of recombinant microbial expression systems, the synthesis of many different polypeptides has been shown to be deleterious to the host cell. As a consequence, there is selective pressure against the expression of such polypeptides, such that the only cells which accumulate in a scale-up of such a recombinant culture are those which have ceased to express the foreign polypeptide or express so little of the foreign polypeptide that the culture becomes an uneconomical source of that polypeptide. In some cases, the deleterious effect is so strong that when expression is driven by a strong constitutive promoter, newly transformed cells fail to propagate and form colonies on selective plates. These deleterious effects can be overcome by using an inducible promoter to direct the synthesis of such polypeptides. A number of inducible genes exist in S. cerevisiae. Four well-characterized inducible genetic systems are the galactose (GAL) utilization genes, the alcohol dehydrogenase 2 (ADH2) gene, the alpha mating factor gene, and the pho5 gene.

S. cerevisiae has 5 genes which encode the enzymes responsible for the utilization of galactose as a carbon source for growth. The GAL1, GAL2, GAL5, GAL7 and GAL10 genes respectively encode galactokinase, galactose permease, the major isozyme of phosphoglucomutase, α-D-galactose-1-phosphate uridyltransferase and uridine diphosphogalactose-4-epimerase. In the absence of galactose, very little expression of these enzymes is detected. If cells are grown on glucose and then galactose is added to the culture, these three enzymes are induced coordinately, by at least 1,000-fold, (except for GAL5, which is induced to about 5 fold) at the level of RNA transcription. The GAL1, GAL2, GAL5, GAL7 and GAL10 genes have been molecularly cloned and sequenced. The regulatory and promoter sequences to the 5' sides of the respective coding regions have been placed adjacent to the coding regions of the lacZ gene. These experiments have defined those promoter and regulatory sequences which are necessary and sufficient for galactose induction.

S. cerevisiae also has 3 genes, each of which encodes an isozyme of ADH. One of these enzymes, ADHII, is responsible for the ability of S. cerevisiae to utilize ethanol as a carbon source during oxidative growth. Expression of the ADH2 gene, which encodes the ADHII isozyme, is catabolite-repressed by glucose, such that there is virtually no transcription of the ADH2 gene during fermentative growth in the presence of glucose levels of 0.1% (w/v). Upon glucose depletion and in the presence of non-repressing carbon sources, transcription of the ADH2 gene is induced 100- to 1000-fold. This gene has been molecularly cloned and sequenced, and those regulatory and promoter sequences which are necessary and sufficient for derepression of transcription have been defined.

Alpha mating factor is a sex pheromone of S. cerevisiae which is required for mating between MATα and MATa cells. This tridecapeptide is expressed as a prepropheromone which is directed into the rough endoplasmic reticulum, glycosylated and proteolytically processed to its final mature form which is secreted from cells. This biochemical pathway has been exploited as an expression strategy for foreign polypeptides. The alpha mating factor gene has been molecularly cloned and its promoter with pre-pro-leader sequence has been utilized to express and secrete a variety of polypeptides. Likewise, the pho5 gene promoter has been shown to be inducible by low phosphate concentrations and this also has utility for physiologically regulated expression of foreign proteins in yeast.

The alpha mating factor promoter is active only in cells which are phenotypically α. There are 4 genetic loci in S. cerevisiae, known as SIR, which synthesize proteins required for the repression of other normally silent copies of a and α information. Temperature-sensitive (ts) lesions which interfere with this repression event exist in the gene product of at least one of these loci. In this mutant, growth at 35°C abrogates repression, resulting in cells phenotypically a/α in which the alpha mating factor promoter is inactive. Upon temperature shift to 23°C, the cells phenotypically revert to α such that the promoter becomes active. The use of strains with a ts SIR lesion has been demonstrated for the controlled expression of several foreign polypeptides.

### OBJECTS OF THE INVENTION

It is an object of the present invention to provide multiple HBV surface proteins simultaneously expressed in a yeast host which form mixed particles composed of two or more HBV proteins. It is another object of the present invention to provide a method of producing in a yeast host, multiple HBV surface proteins which form particles and which contains substantially reduced entrapped carbohydrate content. An additional object of the present invention is to provide a vaccine against HBV comprising the HBV protein particles containing multiple HBV surface proteins with substantially reduced entrapped carbohydrate content for both active and passive treatment of prevention of disease and/or infections caused by HBV or other agents serologically related to HBV. Another object of the present invention is to provide antigens and immunogens for the development of diagnostic reagents for such diseases and/or infections. A further object of the present invention is to provide conditions for the large scale growth of recombinant host cells and the purification of the recombinant multiple HBV surface proteins in particulate form. These and other objects of the present invention will be apparent from the following description.

### SUMMARY OF THE INVENTION

Multiple HBV proteins have been simultaneously expressed at high yield and form particles in a recombinant yeast host which is genetically deficient in its ability to glycosylate proteins or in a yeast host which is wild-type for glycosylation. The expression of multiple HBV surface proteins simultaneously in yeast cells results in the formation of the characteristic particles containing multiple forms of the surface proteins at predetermined ratios. Formation of these particles in a "wild-type" yeast host cell may result in the entrapment of yeast cell substances within the particles. Using "wild-type" yeast host cells substantial amounts of yeast cell carbohydrate may become entrapped within the HBsAg particles. In order to circumvent the production of a HBV vaccine consisting of particles which contain substantial amounts of carbohydrate, the HBV surface proteins may be simultaneously produced and purified from a recombinant yeast host which is genetically deficient in its ability to glycosylate or hypermannosylate proteins. Multiple HBV surface proteins produced by such a host form particles which contain substantially less carbohydrate than particles produced in wild-type yeast cells. These HBV surface protein particles, containing multiple surface protein serotypes and if produced in glycosylation or hypermannosylation deficient yeast also having substantially reduced entrapped carbohydrate content, are useful as a vaccine for the treatment and/or prevention of HBV related infections, and as an antigen for immunologic diagnosis with reduced reactivity with naturally occuring anti-yeast antibodies.

### BRIEF DESCRIPTION OF THE DRAWINGS

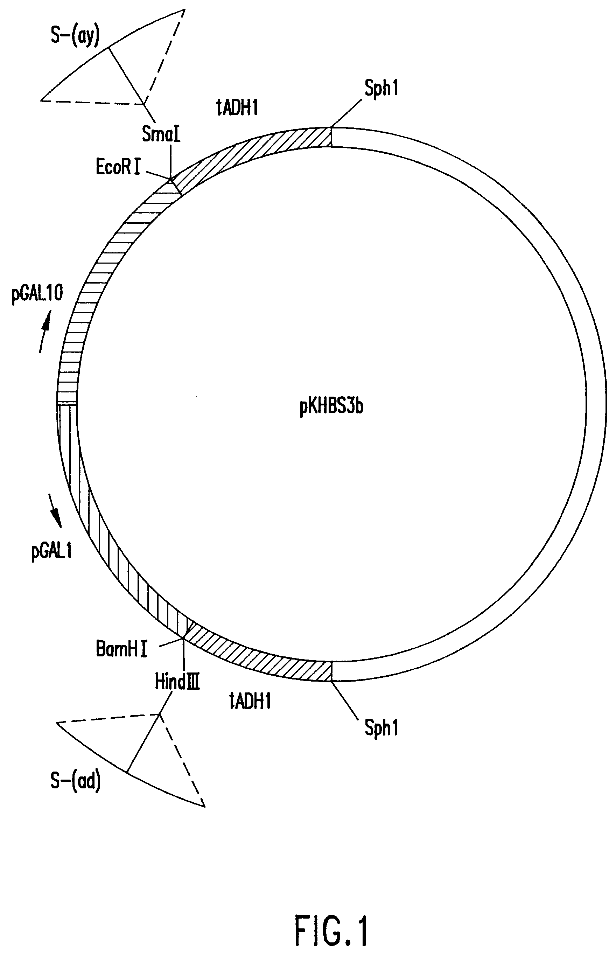

Figure 1 - Figure 1 is a schematic diagram of plasmid pKHBS-3b which contains two HBsAg ORF's, the ay serotype ORE driven by the pGAL10 promoter, and the ad serotype ORF driven by the pGAL1 promoter in the bidirectional promoter vector, with the tADH1 terminators and a LEU2+ selectable marker in a pC1/1 based plasmid.

### DETAILED DESCRIPTION OF THE INVENTION

The present invention is directed to a method for the preparation of two or more HBV proteins which form mixed particles containing the two or more proteins in the same particle, for use as a vaccine against HBV. In addition the invention is directed to the such mixed particles containing substantially reduced carbohydrate content. In order to develop an HBV vaccine candidate for protection of individuals who become exposed to HBV with mutations in the "a" epitope or who fail to respond to the "a" epitope in current vaccines by expressing a mixed-particle antigen displaying both the ay and ad serotypes of HBsAg in particulate form a coexpression strategy was developed. Expression was achieved in yeast hosts which contain the mnn9- mutation which prevents hyperglycosylation and co-purification of entrapped yeast carbohydrate as well as in wild-type yeast host cells. It is readily apparent to those skilled in the art that expression in yeast hosts with mutations in glycosylation related genes, other than mnn9, would also result in glycosylation modification.

Co-expressing the HBsAg adw and ayw serotypes to produce particles formed of both serotypes of S antigen was achieved by putting two HBV genes on one plasmid under the control of two separate promoters in a bidirectional vector. Based on approximate expected relative promoter strengths the ratio of the expressed polypeptides in this transformant is about 1:1. The polypeptides co-assemble into particles which display the ad as well as the ay serotypes of HBsAg.

Dane particles (serotype adw and ayw) were utilized as the source of HBV nucleic acid for the isolation of the viral ORFs. It is readily apparent to those skilled in the art that this invention extends to the use of nucleic acid from HBV strains or related viruses with other serologic reactivities (including but not limited to ayr, ayw, adr, adw and other epitope variants) which derive from viral genetic diversity. The endogenous polymerase reaction was employed in order to produce covalently-closed circular double-stranded DNA of the HBV genome from the nicked and gapped nucleic acid form that natively resides in the HB virion. The DNA was isolated, digested to completion with EcoRI, and cloned into the EcoRI site of pBR322, thus generating pHBV/ADW-1, or pHBV/AYW-1. The recombinant plasmids containing the HBV genome in a circularly permuted form at the EcoRI site of the PreS region were selected. The complete ORF of the adw serotype encoding the 55 amino acids of the preS2 region, and the 226 amino acids of the S region was constructed first by purifying the 0.8 kilobase pair (kbp) fragment obtained following digestion of pHBV/ADW-1 with EcoRI and AccI; this fragment encodes the preS2+S polypeptide lacking only the initiation codon, the amino-terminal 3 amino acids, the carboxy-terminal 3 amino acids, and the translational terminator codon.

Oligonucleotides were synthesized and ligated to this fragment, converting it to a HindIII fragment containing a 10 bp yeast-derived non-translated 5' flanking sequence and the complete preS2+S ORF was chosen such that the termination codon was directly joined to a natural HindIII site in the ADH1 transcriptional terminator, thus creating a completely native yeast-derived junction without any additional intervening bases. It is readily apparent to those skilled in the art that for expression of HBV surface and related proteins, any suitable yeast-active transcriptional terminator may be substituted for ADH1.

The 5' flanking sequence for the construction 
ACAAAACAAA (SEQIDNO: 1)
1         10
was chosen to correspond to that for the non-translated leader (NTL) of the yeast gene GAP63 (GAP) [Holland, J. Biol. Chem., 225, 2596 (1980)] and is also a consensus for the GAP gene family. The construction was made in such manner as to join the NTL directly to the initiation codon of the envelope ORF without the intervention of any additional bases. Therefore, it is readily apparent to those skilled in the art that, for expression of HBV surface proteins, the selection of NTL sequences extends to other sequences which result in suitable expression levels.

DNA sequence analysis revealed 2 base substitutions which resulted in amino acid differences from the preS2+S sequence encoded by the DNA of pHBpreSGAP347/19T [Valenzuela et al., Biotechnology, 3(4), 317-320 (1985)]. In order to evaluate identical polypeptides for both constructions, these nucleotide substitutions, which were T instead of C at base 64 of the 846 bp ORF of HBV preS2+S (encoding Phe rather than Leu) and C instead of A at base 352 (encoding His rather than Gln) were changed by site-directed mutagenesis [Zoller et al., Nucleic Acids Research 10:6487-6500 (1982)]. The encoded amino acid sequence for the optimized construction then was verified. It is readily apparent to those skilled in the art that this invention is not limited to this sequence and extends to any sequence wherein the DNA encodes a polypeptide with HBV-related antigenicity.

The large DNA fragment of 3.3kbp which contains pUC19 and the HBsAg coding region was separated from the preS2 encoding DNA fragment after digestion with EcoRI and StyI, and purified by preparative agarose gel electrophoresis. A synthetic DNA oligonucleotide was then ligated with the pUC19-HBsAg fragment. This synthetic DNA oligonucleotide contains 5' EcoRI and 3' StyI sticky ends as well as providing a HindIII site immediately following the 5' EcoRI site. In addition, the synthetic DNA oligonucleotide contains the HBsAg ATG codon plus the 9 upstream nucleotides and the 21 downstream nucleotides including the StyI site.

This oligonucleotide rebuilds the complete coding region of the HBsAg and allows its subsequent removal intact, from the pUC19 based vector by digestion with HindIII.

The HBsAg ayw serotype DNA was constructed in a similar manner as the adw serotype described above.

The pUC19-HBsAg DNA fragments (serotypes ad and ay) with the ligated synthetic DNA oligonucleotide described above were used to transform E. coli. Recombinant plasmids were selected which possess the complete reconstructed HBsAg coding regions. The complete HBsAg open reading frames (ORF) were removed from the recombinant plasmids by digestion with HindIII followed by isolation and purification of the 0.7kbp HBsAg DNAs by preparative agarose gel electrophoresis for cloning into the bidirectional GAL1GAL10 promoter expression vector.

Construction of the bidirectional inducible vector expressing both serotypes of S antigen was done as follows.

Plasmid pBM272(which contains the GAL1/GAL10 promoter fragment in YCP50) [M. Johnston and R.W. Davis (1984) Mol. Cell. Biol. 4, pp.1440-1448; G. Barnes and J. Rine (1985) PNAS 82, pp.1354-1358] was digested with restriction endonucleases HindIII and SphI and was ligated with a 0.35kbp DNA fragment with HindIII-SphI ends containing the tADH1 terminator. This plasmid was used to transform E. coli and transformants were screened for the plasmid containing the tADH1 terminator. This plasmid was digested with EcoRI and SalI, and the 1.14kbp DNA fragment containing the pGAL1pGAL10 promoter region with the tADH1 terminator downstream from the GAL1 promoter was isolated and purified by preparative agarose gel electrophoresis.

Plasmid pUC18GAL10tADH1 was digested with HindIII and EcoRI to remove the GAL10 promoter region and the large vector fragment was agarose gel purified. Two synthetic DNA oligonucleotides were synthesized with have the following sequence: 
AATTGTCGAC AGCTAGCTGA ATTCCCGGG (SEQIDNO: 10)
1         10         20        29
AGCTCCCGGG AATTCAGCTA GCTGTCGAC (SEQIDNO: 11).
1         10         20        29
These two oligonucleotides were annealed and ligated to the HindIII-EcoRI digested pUC18GAL10tADH1 vector. The synthetic oligonucleotide described above, when ligated to the pUC18GAL10tADH1 vector as described, destroys the EcoRI and HindIII sites of the vector, and adds SalI, EcoRI and SmaI recognition sites to the vector.

The vector containing the ligated synthetic oligonucleotide was digested with SalI and EcoRI and the vector was purified by agarose gel electrophoresis. Into this vector was ligated the SalI-EcoRI DNA fragment containing the tADH1 terminator downstream from the GAL1 promoter, plus the GAL10 promoter. This ligated vector was used to transform E. coli and transformants were screened for the presence of the appropriate plasmid construction. The resulting plasmid contains the pGAL1pGAL10 promoter region with a tADH1 terminator downstream for each promoter. Cloning sites between the GAL10 promoter and the tADH1 terminator include EcoRI and SmaI, and cloning sites between the GAL1 promoter and the tADH1 terminator include BamHI and HindIII.

This construction takes advantage of the bidirectionally divergent yeast pGAL1pGAL10 promoter region. Unique cloning sites between these promoters and added ADH1 sequences were engineered. The S (adw), and S (ayw), ORFs were inserted at these unique sites.

The ayw serotype ORF was obtained by digestion with HindIII and agarose gel purification of the 0.7 kbp DNA fragment. This fragment was blunt ended and ligated into the SmaI site of the bidirectional inducible vector pKHBS-3 downstream from the GAL10 promoter. The ligated vector was used to transform E. coli and transformants were screened for the appropriate plasmid containing the ayw serotype ORF in the correct orientation.

The HBsAg adw serotype ORF was obtained from plasmid pUCHBsAg by digestion with HindIII, and agarose gel purification of the 0.7kbp DNA fragment. This fragment was ligated into the HindIII site of the bidirectional inducible vector (containing the ayw serotype ORF) downstream from the GAL1 promoter.

The ligated vector was used to transform E. coli and transformants were screened for the appropriate plasmid containing both HBsAg ORFs in the correct orientation. The final plasmid construction is shown in Figure 1.

This bidirectional vector was used to coexpress the ORFs for S(adw), and S(ayw) from pGAL1 and pGAL10 respectively. Yeast were transformed, and cloned seeds established.

Parental strain CF52 and CF55 were obtained as follows: The α mating type strain CZ5/LB347-1C (mnn9⁻, Suc²⁻) was mated with the "a" type strain 2150-2-3 (leu2⁻, ade1⁻) by mixing the strains on a YEHD complete media plate. To select for diploids, the mated strains were replica plated onto leu⁻ minimal medium containing 2% sucrose as the sole carbon source. After isolating single colonies, the diploids were sporulated, and asci were dissected by standard techniques. The KHY-107 strain was isolated as a single spore and characterized as cir⁺, ade1⁺, leu2⁻, and mnn9- (by Schiff stain technique). KHY107 (cir 0) was derived from strain KHY107 (cir⁺) as described by Broach [Methods in Enzymology, 101, Part C, pp 307-325, (1983)]. The cured strain was made ura3⁻ by integrating a disrupted ura3 gene. The resulting strain, KHY-107ura3Δ, was grown in rich media to allow the accumulation of spontaneous mutations and a canavanine resistant mutant was selected. The mutant strain, CF55, was shown by complementation tests to be can1⁻. The GAL10pGAL4 expression cassette was integrated into the HIS3 gene of CF55 (Methods in Enzymology, 1990, 185 pp297-309) to yield the final host strain CF52 (Mata leu2-2,112 mnn9⁻ ura3Δ can1 his3Δ::GAL10pGAL4-URA3, cir°). The strains CF52 and CF55 were established as frozen stocks for evaluation and subsequent transformation or experimentation.

Recombinant yeast from the frozen stocks was grown in YEHD medium [Carty et al., J. Industrial Micro., 2, 117-121, (1987)]. After growth to stationary phase, yeast cells were harvested. Lysates were prepared, resolved by sodium dodecylsulfate-polyacrylamide gel electrophoresis (SDS-PAGE), and immunoblotted with antibodies to HBsAg. One polypeptide was found with molecular weight of about 24-kD in accord with the predicted molecular weight of the translational product of the S ORF. Furthermore, lysates of recombinant, but not parental, yeast were positive for S by radioimmunoassay (Ausria^{R}).

The bidirectional yeast-derived pGAL1pGAL10 promoter region initiates transcription of the HBsAg and other genes. Therefore, it is readily apparent to those skilled in the art that any bidirectional inducible yeast-active promoter sequence may be substituted for the pGAL1pGAL10 promoter region. It is also readily apparent to those skilled in the art that a suitable assay system, e.g., immunoblot or RIA or enzyme-linked immunoassay (EIA), should be utilized in order to assay expression of HBsAg and related polypeptides in this system, such that the time of harvesting of the culture for attaining a maximal yield can be optimized.

It is readily apparent to those skilled in the art that the selection of a suitable yeast strain for expression of HBV surface proteins encompasses a wide variety of candidates. Suitable yeast strains include but are not limited to those with genetic and phenotypic characteristics such as protease deficiencies, and altered glycosylation capabilities.

In order to control and define the glycosylation of recombinant yeast-expressed HBV proteins, S. cerevisiae strain CF52 (Mata leu2-2, 112 ura3Δ can1 his3Δ:: GAL10pGAL4-URA3, cir°, mnn9⁻) was constructed as described above.

The bidirectional inducible expression plasmid containing two HBsAg ORF's of different serotype was used to transform CF52 (Mata leu2-2, 112 ura3Δ can1 his3Δ:: GAL10pGAL4-URA3, cir°, mnn9-). Transformed clones were selected on minimal medium (leu-) containing 1M sorbitol. These cloned transformants were established as frozen stocks in 17% glycerol for subsequent evaluation and further experimentation.

To produce a glycosylation wild-type control the expression plasmid is also used to transform yeast strain CF54, which is isolated by standard techniques from strain CF52 and which is a spontaneous revertant to MNN9+ (and thus is wild-type for glycosylation but otherwise of identical genotype to strain CF52). Transformed clonal isolates are established as frozen stocks in 17% glycerol for subsequent evaluation and further experimentation.

Clones of transformed yeast containing the expression plasmids were plated onto leu⁻ selective agar plates (containing 1M sorbitol for mnn9⁻ transformants) and incubated at 30°C for 2-3 days. These yeast were inoculated into 5-7 mL cultures of complex YEHD (Carty et al., supra) medium (containing 0.1M to 1M sorbitol) and the cultures were incubated at 30°C with aeration for 12-18 hours. Flasks containing 50 mL complex YEHD media with 0.1M sorbitol (hereafter called YEHDS), plus 2% galactose, were inoculated from the above cultures (to an inital A₆₀₀ = 0.1) and were incubated at 30°C with shaking (350 rpm) for 48-72 hours to a final A₆₀₀ of 10-16. Samples of 10 A₆₀₀ units were dispensed into tubes, and the yeast cells were pelleted by centrifugation at 2000xg for 10 minutes. Samples either were assayed directly or stored frozen at -70°C. At the time of assay, the pellets were resuspended in 0.3 mL of phosphate- buffered saline (PBS) containing 2mM phenylmethyl sulfonyl fluoride (PMSF) and transferred to 1.5 mL Eppendorf tubes. Yeast cells were broken by: 1) the addition of 200-300 mg of washed glass beads (0.45 mm) and agitation on a vortex mixer for 15 minutes, 2) addition of TRITON X-100 to 0.5%, 3) agitation on the vortex mixer for 2 minutes, and 4) incubation at 4°C for 10 minutes. Cellular debris and glass beads were removed and the supernatant assayed for protein [by the method of Lowry et al., J. Biol. Chem., 193, 265, (1951)] and S (AUSRIA^{R}).

Immunoblot analysis with anit-p24 sera of the polypeptide derived from the recombinant clones described above, in host cells with the mnn9⁻ phenotype, showed one band with apparent molecular size of about 24-kD which is the size predicted for the translation product of the S ORF).

For recombinant proteins, the qualitative and quantitative glycosylation patterns are a function of and largely dependent upon the host cell species, and within a species upon the cell line. It is thus readily apparent to those skilled in the art that the selection of a host strain extends to species and cell lines other than S. cerevisiae for which mutations in enzymes in the glycosylation pathway may be identified. It is also readily apparent to those skilled in the art that selection of host strains of S. cerevisiae extends to all strains in which mutations in enzymes of the glycosylation pathway may be identifed.

The transformed clones were then screened for the presence of the HBsAg DNA and expression of p24. Cells were grown in YEHDS medium also containing galactose for the GAL10 promoter plasmids to induce expression following glucose depletion. Lysates were prepared, resolved by sodium dodecyl sulfate- polyacrylamide gel electrophoresis (SDS-PAGE) and Western blotted to nitrocellulose. A p24 product was found to be specific to envelope protein by virtue of its presence only in induced transformants and its reactivity with anti-p24(S) serum. One clone was selected for further analysis. Furthermore, lysates of transformants, but no parental S. cerevisiae, was positive for HBsAg, by radioimmunoassay or by immunoblot.

This bidirectional vector was used to coexpress the ORFs for S(adw) and S(ayw) from pGAL1 and pGAL10 respectively. Yeast cells were transformed, and cloned seeds established.

An immune-affinity column, bound with goat antibodies which recognize the particulate form of HBsAg, has been utilized to purify S and S-related proteins from transformed S. cerevisiae. The eluted product is positive for HBsAg by radioimmunoassay, and is of particulate form in electron microscopy. Such a particulate form which contains HBsAg is effective as a HBV vaccine and diagnostic reagent.

Yeast cells transformed with expression vectors coding for a hepatitis B virus surface protein or variants thereof are grown and harvested. The cells may be stored if desired by washing the cells in a buffer solution, e.g. PBS, and forming a cell paste which is typically stored frozen at -70°C.

Purification of HBsAg and related proteins typically begins as follows. A batch of fresh or frozen cell paste is suspended in a buffer, preferably TRIS, at a high pH ranging between about 8.5 and about 11.0, preferably about 10.5 (the buffer may also contain suitable protease inhibitors). The cells are then disrupted, preferably by mechanical means. The gentle bead breakage method of disruption has been found to be unsuitable for scale-up use. Disruption by a high pressure homogenizer (about 10,000 to 20,000psi, using a Gaulin or Stansted homogenizer) is preferred because of its rapid and efficient operation.

Disruption of the yeast cells results in a crude extract. The crude extract is then pH adjusted. The pH is adjusted to within the range of 8.0 to 11.0, with 10.5 being preferred.

It may be desired at this point to add a detergent to the crude extract. The addition of a detergent will facilitate the separation of yeast cell membranes from unwanted cellular debris. It has been shown that preS1+preS2+S protein, as well as other forms of the surface proteins, may associate with yeast cell membranes. A variety of neutral or non-ionic detergents can be used, including but not limited to detergents of the TRITON-N series, TRITON-X series, BRIJ series, TWEEN series or EMASOL series, deoxycholate, octylglucopyranoside or NONIDET-NP-40. Zwitterionic detergents such as CHAPS or CHAPSO are also useful and suitable agents.

If a detergent is to be used, the preferred detergent is TRITON X-100 at a concentration of about 0.5%. It must be stressed that the method of this invention does not require detergent use at this step and the use of detergents is optional.

The extract then may be heat treated if protease inhibitors were not added during lysis Heat treatment is effective over a range of temperatures and for a range of treatment duration. Typically a temperature range of 45°C to 60°C is used, with 50°C as the preferred temperature. The duration of heat treatment typically ranges between 20 to 40 minutes with 30 minutes as the preferred time. The extract is heat treated in a suitable vessel and the vessel is immersed in a heated bath, or a heat exchanger is used. The material is then cooled to about 10°C, preferably by placing it into an ice-water bath or by using a heat exchanger. It will be readily apparent to those skilled in the art that, according to the method of this invention, the order in which the heat treatment and the debris removal steps are done may be reversed without significant effect on the result of this procedure. Alternatively, whole yeast cells can be heated in a neutral pH buffer, disrupted and detergent added as described above.

Removal of cellular debris from the heat treated crude extract is necessary to prevent physical occlusion during subsequent purification steps. Debris can be removed by centrifugation, microfiltration, or filtration producing a clarified extract. Centrifugation and microfiltration are the most preferred methods. Centrifugation can be done for varying lengths of time at different centrifugal forces. Centrifugation at about 3,000 x g for 15 minutes at 4°C has been found adequate. It may also be advantageous to dilute the extract before centrifugation to reduce the typically viscous nature of a crude yeast cell extract. Dilution will not alter any subsequent steps of this procedure.

Microfiltration has an advantage in that filtration and dialysis can be performed simultaneously. Several types of microfiltration units are suitable for use in this step, e.g. KROSFLO by Microgon Inc. or any variety of hollow fiber cartridges by Amicon or A/G Technology. The preferred microfiltration method is to pass the extract through Prostak Durapore (Millipore) membrane, plate and frame microfiltration unit with a pore size of about 0.1 microns to 0.2 microns, at an inlet pressure of about 2 to 7 psi, using a buffer consisting of about 0.1M TRIS, pH about 10.4 and about 0.1% TRITON X-100.

The supernatant from centrifugation or the filtrate from microfiltration may be concentrated prior to the next step of this procedure. Concentration can be achieved by several methods including, but not limited to, dialysis, filtration, lyophilization, ultrafiltration and diafiltration. The preferred method of concentration of the present invention is to run the clarified extract through a 10⁵ molecular weight cut off, hollow fiber ultrafiltration system. The volume of the clarified extract is typically reduced by about 6.5 fold for the microfiltration product and about 2 fold for the diluted, centrifuged product, yielding a concentrated retentate. Following concentration, the retentate is diafiltered to further remove lower molecular weight contaminants. Diafiltration is performed using a 10⁵ molecular weight cutoff, hollow fiber system.

If TRITON X-100 was added, it can be removed by several conventional methods including, but not limited to, dialysis, addition of certain organic solvents, refrigeration, chromatographic separation, and contact with a gel or resin which specifically binds detergents, such as Extractogel (Pierce) and XAD resin (Romicon). The preferred method of this invention to remove TRITON X-100 is to circulate the heat treated extract containing TRITON X-100 through a cartridge of XAD-2 or XAD-4 resin (polystyrene divinylbenzene). The heat treated extract is circulated through the XAD cartridge for about 10 hours at 4°C and then collected in a suitable vessel, for example, a sealable glass bottle.

If the cells were disrupted in a high pH buffer, the pH of the heat treated extract is then adjusted to between about pH 7.0 to about 7.9 with the preferred pH of about 7.7. Adjusting the pH to about 7.7 following heat treatment at a high pH according to the method of this invention, greatly facilitates the adsorption of envelope proteins to the wide pore silica utilized in a subsequent step. Adjustment of the pH of the heat treated extract can be performed prior to the TRITON X-100 removal step without effecting the outcome of the procedure. Therefore, it will be obvious to those skilled in the art that, according to the method of this invention, the order in which the pH adjustment and the TRITON X-100 removal steps are done may be reversed without significant effect on the result of this procedure.

The HBsAg is then easily separated from the contaminants yielding substantially purified particles. The preferred method of eliminating the contaminants is to adsorb the particles onto wide pore silica. The most preferred method of this invention is to adsorb the surface protein onto a wide pore silica with a pore size range of about 1000 to 1500 angstroms and silica particle size range of about 30 to 130 microns (Amicon). The surface protein readily enters the pores of the silica and is retained. The yeast cellular protein contaminants can therefore be easily washed away.

Adsorption of particles onto wide pore silica can be done chromatographically or in a non-chromatographic, batchwise fashion. Chromatographic adsorption is done by passing the pH adjusted extract through a bed of wide pore silica in a column chromatography apparatus. Typically, about one liter of heat treated extract is applied to a 5 cm jacketted column apparatus containing about 300 ml (about 100 g dry weight) of wide pore silica beads at a flow rate of about 200ml/hour.

Non-chromatographic adsorption onto wide pore silica is typically done by mixing the heat treated extract with the silica in a suitable vessel, e.g. a sealable glass bottle. The preferred method is to add 300 ml of wide pore silica to about one liter of heat treated extract in a glass bottle and incubate with constant mixing. Adsorption preferrably continues for about 1.5 hours at about 4 - 8°C although different times and temperatures are suitable.

Washing of the surface protein-adsorbed silica free of unadsorbed material can also be done non-chromatographically, or the silica can be poured into a column apparatus, as previously described, for chromatographic adsorption. Batchwise washing is done by draining the heat treated extract from the wide pore silica and adding several volumes of a buffer which will not cause the release of particles adsorbed onto the silica. The preferred buffer is PBS. The silica is drained and the washing steps are repeated 3 to 5 times.

Chromatographic washing of the surface HBsAg-adsorbed silica is done by passing PBS through the silica at a flow rate of about 200ml/hour until the extinction at 280nm is constant.

The HBsAg is eluted from the washed wide pore silica using a buffer solution with a pH between about 8.5 to 9.0. Surface proteins are preferably desorbed using a buffer solution consisting of about 0.05 M Borate at a pH of about 8.7. Desorption of HBsAg proteins can be facilitated at elevated temperatures over a wide range. Desorption at about 55°C is preferred.

Non-chromatographic desorption is done by mixing 1200 ml of 0.05 M Borate buffer at pH 8.7 with about 700 ml of washed particle-adsorbed wide pore silica. Desorption continues for about 25 minutes. The eluate is then collected, the desorption steps are repeated twice and the eluate is cooled.

Chromatographic desorption is done by warming the jacketted column of washed silica to about 55°C. The 0.05M Borate buffer at pH 8.7 is warmed to 55°C and then applied to the column at a rate of 500 ml/hour. The eluate is then collected and cooled. The volume of eluate is usually roughly equivalent to the volume of heat treated extract applied to the wide pore silica.

Concentration of the eluted particles is usually desired. The preferred concentration method is to pass the eluate through a 10⁵ molecular weight cut-off hollow fiber diafiltration system using a 0.05M Borate buffer, pH 8.7. The volume of the eluted surface protein may be generally reduced by as much as 16 fold using this system. The diafiltration retentate can be sterilized by microfiltration if necessary.

HBsAg can be further purified using an immune-affinity column. An immun-affinity column bound with goat antibodies which recognize the particulate form of HBsAg, has been utilized to purify HBsAg containing particles from transformed S. cerevisiae [U.S. Patent 4,816,564], and as described above.

The carbohydrate content of the particles is determined by the method of Dubois, M. et al., Anal. Chem., 28, pp.350, 1956. The general principle of this procedure is that simple sugars, oligosaccharides, polysaccharides and their derivatives, including the methyl ethers with free or potentially free reducing groups, give an orange yellow color when treated with phenol and concentrated sulfuric acid. The amount of color produced at a constant phenol concentration is proportional to the amount of sugar present.

To determine the carbohydrate content of a sample of HBV surface proteins, 1 mL of a solution containing between 10 to 70 µg of protein is placed in a test tube. A series of carbohydrate standards and blank samples are prepared. One mL of a 5% phenol solution is added to each tube, the tubes are mixed, and 5 mL of a 96% sulfuric acid solution is added and mixed. The tubes are incubated at room temperature for 10 minutes, mixed, and incubated at 25 to 30°C for 20 minutes. The samples are read in spectrophotometer (A₄₉₀ for hexoses and methylated hexoses, and A₄₈₀ for pentoses, uronic acid, and their methylated derivatives) and the amount of carbohydrate in the HBsAg samples is determined by comparison with the carbohydrate standards.

HBsAg produced in "wild-type" recombinant yeast cells (CF54), and HBsAg produced in the CF52 recombinant yeast cells are both analyzed for carbohydrate content as described above. Based on these results, a ratio of the amount of carbohydrate to protein present in each sample is calculated by dividing the micrograms of carbohydrate by the micrograms of protein in the sample. This ratio calculation demonstrates that HBsAg produced in mnn9-recombinant yeast cells consistently contains one tenth of the carbohydrate content of HBsAg produced in recombinant "wild-type" yeast cells. These results will show that HBsAg produced in the mnn9-mutant yeast cells contains substantially reduced amounts of carbohydrate when compared to HBsAg produced in "wild-type" yeast cells.

The following examples illustrate the present invention without, however, limiting the same thereto. The disclosure of each reference mentioned in the following examples is hereby incorporated by reference.

### EXAMPLE 1

### Cloning of HBV DNA in pBR322

HBV Dane particles (serotype adw and ayw) were isolated and purified from human plasma (carrier), and double-stranded DNA was synthesized by the endogenous polymerase in the Dane particles according to the methods of Landers et al., [J. Virology, 23, 368-376, (1977)] and Hruska et al., [J. Virology, 21, (1977)]. The DNA was isolated after digestion with Proteinase K in SDS followed by extraction with phenol/chloroform and ethanol precipitation. The HBV genomic DNA was digested with EcoRI, producing a single 3.2 kbp fragment, that was cloned into the EcoRI site of pBR322 to form pHBV/ADW-1 or pHBV/AYW-1. The presence of the HBV DNA was confirmed by EcoRI digestion, Southern blot transfer to nitrocellulose, and hybridization with [³²P]-labelled specific oligonucleotide probes.

### EXAMPLE 2

### Cloning of the preS2+S and S Genes, serotype adw, into Cloning Vectors

Plasmid pHBV/ADW-1 (described in Example 1) was digested with EcoRI and AccI, and the 0.8 kbp fragment was purified by preparative agarose gel electrophoresis. Also, a pUC plasmid was digested with EcoRI and BamHI and the linear vector was purified by preparative agarose gel electrophoresis.

To reconstruct the 5' portion of the preS2+S ORF, a pair of oligonucleotides was synthesized which reconstitutes the ORF from the EcoRI site upstream to the ATG through a 10 bp NTL sequence through a HindIII site to an EcoRI terminus. The sequence of these oligonucleotides are:

AATTCAAGCT TACAAAACAA AATGCAGTGG (SEQIDNO: 2)
1 10 20 30

GTTCGAATGT TTTGTTTTAC GTCACCTTAA (SEQIDNO: 3)
1 10 20 30

To reconstitute the 3' portion of the preS2+S ORF, a second pair of oligonucleotides was synthesized which reconstitutes the ORF from the AccI site through the translational terminator through a HindIII site to a BamHI terminus. The sequence of these oligonucleotides are:

ATACATTTAA AGCTTG (SEQIDNO: 4)
1         10     16

TGTAAATTTC GAACCTAG (SEQIDNO: 5)
1 10 18

The oligonucleotide pairs were annealed, and then ligated to the pUC EcoRI - BamHI digested vector. The resultant vector (2.8kbp) was purified by preparative agarose gel electrophoresis. The 0.8kbp EcoRI - AccI fragment from above was ligated with this vector. The presence and orientation of the PreS2+S ORF was confirmed by restriction endonuclease analysis and Southern blot. DNA sequence analysis [Sanger et al., 1977] revealed two base substitutions that resulted in amino acid differences from the sequence encoded by the DNA of plasmid HBpreSGAP347/19T. To evaluate identical polypeptides for both constructions, these substitutions, which were T instead of C at base 64 (encoding Phe rather than Leu) and C instead of A at base 352 (encoding His rather than Gln), were changed by site-directed mutagenesis. [Zoller and Smith 1982, Nucleic Acids Research, 10, pp6487-6500].

A plasmid containing the HBsAg coding region without the preS2 coding region was constructed as follows: The pUCHBpreS2+S plasmid (described above) was digested with EcoRI and StyI restriction endonucleases. The large DNA fragment (3.3kbp) which contains pUC and the HBsAg coding region was separated from the preS2 encoding DNA fragment and purified by preparative agarose gel electrophoresis. A synthetic DNA oligonucleotide pair: 
AATTCAAGCT TACAAAACAA AATGGAGAAC ATCACATCAG
1         10          20          30          40
GATTC (SEQIDNO: 6)
    45
GTTCGAATGT TTTGTTTTAC CTCTTGTAGT GTAGTCCTAA
1         10          20          30          40
GGATC (SEQIDNO: 7)
    45
was annealed and then litagated with the pUCHBsAg fragment. This synthetic oligonucleotide pair contains 5' EcoRI and 3' StyI sticky ends as well as providing a HindIII site immediately following the 5' EcoRI site. In addition, the synthetic DNA oligonucleotide pair contains the HBsAg ATG codon, the upstream 10bp non-translated leader sequence, and the 21 downstream nucleotides including the StyI site.

This oligonucleotide pair rebuilds the complete coding region of the HBsAg and allows its subsequent removal intact, from the pUC based vector by digestion with HindIII.

The pUC-HBs(ad)Ag DNA vector with the ligated DNA oligonucleotide pair described above was used to transform E. coli. Recombinant plasmids were selected which possess the complete reconstructed HBsAg coding region. The complete HBsAg open reading frame (ORF) was removed from the recombinant plasmid by digestion with HindIII followed by isolation and purification of the (0.7kbp) HBsAg DNA by preparative agarose gel electrophoresis for cloning into an expression vector.

### EXAMPLE 3

### Cloning of S(ayw) Gene into cloning vectors

Plasmid pHBV/AYW-1 (described in Example 1) was digested with EcoRI and AccI,, and the 0.82kbp fragment was purified by preparative gel electrophoresis. This fragment was then digested with HincII and the 0.6 kbp DNA fragment was purified by preparative agarose gel electrophoresis. Also, a pUC plasmid was digested with HindIII and BamHI and the linearized vector was purified by agarose gel electrophoresis.

To reconstruct the 5' portion of the S ORF, a pair of oligonucleotides were synthesized which reconstitute the ORF from the HincII site upstream to the ATG through a 10bp NTL sequence to a HindIII terminus. The sequences of these oligonucleotides are:

AGCTTACAAA ACAAAATGGA GAACATCACA TCAGGATTCC TAGGGCCCCT
1 10 20 30 40 50
TCTCGTGTTA CAGGCCGGCT TTTTCTTGTT (SEQIDNO: 8)
60 70 80

AACAAGAAAA AGCCGGCCTG TAACACGAGA AGGGGCCCTA GGAATCCTGA
1 10 20 30 40 50
TGTGATGTTC TCCATTTTGT TTTGTA (SEQIDNO: 9)
60 70 76

To reconstitute the 3' portion of the S ORF, a second pair of oligonucleotides was synthesized. These oligonucleotides are the same as those used to reconstitute the 3' portion of the PreS2+S ORF described in Example 2.

The oligonucleotide pairs were annealed and then ligated to the pUC HindIII - BamHI digested vector. The resulting vector (2.8 kbp) was purified by gel electrophoresis. The 0.6kbp HincII - AccI DNA fragment from above was ligated with this vector. The presence and orientation of S was confirmed by restriction endonuclease analysis and Southern blot.

The complete HBsAg ORF was removed from the recombinant plasmid, [pUCHBs(ay)Ag] by HindIII digestion followed by isolation and purification of the 0.7 kbp HBsAg DNA by preparative agarose gel electrophoresis for cloning into expression vectors.

### EXAMPLE 4

### Construction of the Bidirectional Vector Expressing the HBsAg ORFs Encoding Multiple Serotypes

Plasmid pBM272 which contains the GAL1/GAL10 promoter in YCP50 [M. Johnston and R.W. Davis (1984) Mol. Cell. Giol. 4, pp.1440-1448; G. Barnes and J. Rine (1985) PNAS 82, pp.1354-1358] was digested with restriction endonucleases HindIII and SphI and was ligated with a 0.4kb DNA fragment with HindIII and SphI ends containing the tADH1 terminator. This ligated plasmid was used to transform E. coli and transformants were screened for the plasmid containing the tADH1 terminator. This plasmid was digested with EcoRI and SalI, and the 1.1kb DNA fragment containing the GAL1/GAL10 promoter region with the tADH1 terminator downstream from the GAL1 promoter was isolated and purified by preparative agarose gel electrophoresis.

Plasmid pUC18GAL10tADH1 was digested with HindIII and EcoRI to remove the GAL10 promoter region and the large vector fragment was agarose gel purified. The synthetic DNA oligonucleotides were synthesized which have the following sequence:

AATTGTCGAC AGCTAGCTGA ATTCCCGGG (SEQIDNO: 10)
1         10         20        29

AGCTCCCGGG AATTCAGCTA GCTGTCGAC (SEQIDNO: 11)
1         10         20        29

These two oligonucleotides were annealed and ligated to the HindIII - EcoRI digested pUC18GAL10tADH1 vector. The synthetic oligonucleotide described above, when ligated to the pUC18GAL10tADH1 vector as described, destroys the EcoRI and HindIII sites of the vector, and adds SalI, EcoRI and SmaI recognition sites to the vector.

The vector containing the ligated synthetic oligonculeotide was digested with SalI and EcoRI and the vector was purified by agarose gel electrophoresis. Into this vector was ligated the SalI-EcoRI DNA fragment containing the tADH1 terminator downstream from the GAL1 promoter, plus the GAL10 promoter. This ligated vector was used to transform E. coli and transformants were screened for the presence of the appropriate plasmid construction. The resulting plasmid contains the GAL1/GAL10 promoter region with a tADH1 terminator downstream for each promoter. Cloning sites between the GAL10 promoter and the tADH1 terminator are EcoRI and SmaI, and cloning sites between the GAL1 promoter and the tADH1 terminator are BamHI and HindIII.

The ayw serotype HBsAg ORF was obtained from plasmid pUCHBs(ay)Ag by digestion with HindIII, and agarose gel purification of the 0.7 kbp DNA fragment. This DNA fragment was blunted by use of the DNA polymerase I Klenow fragment. This blunt ended fragment was ligated into the SmaI site of the bidirectional vector downstream from the GAL10 promoter. The ligated vector was used to transform E. coli and transformants were screened for the appropriate plasmid containing the HBsAg ay serotype ORF in the correct orientation.

The HBsAg serotype ad ORF was obtained from plasmid pUCHBs(ad)Ag by digestion with HindIII and agarose gel purification of the 0.7kbp DNA fragment. This fragment was ligated into the HindIII site of the bidirectional vector (containing the ay serotype ORF) downstream from the GAL1 promoter.

The ligated vector was used to transform E. coli and transformants were screened for the appropriate plasmid containing both the HBsAg adw serotype ORF and the HBsAg ayw serotype ORF in the correct orientation. The expression cassette containing the two HBsAg ORF's and the pGAL1/pGAL10 promoter region, and the tADH1 terminators was digested with SphI and the 2.8 kbp DNA was isolated and purified by agarose gel electrophoresis. The DNA was ligated between the SphI sites of the pCl/l shuttle vector. The final plasmid construction is shown in Figure 1.

### EXAMPLE 5

Construction of yeast S. Cerevisiae CF52 (mnn9⁻) mutant
yeast strain

Yeast S. cerevisiae strain KHY 107 (cir⁺, ade1⁺ leu2⁻ and mnn9⁻) was constructed as follows: The α mating type strain CZ5/LB347-1C (mnn9⁻, Suc²⁻) was mated with the "a" type strain 2150-2-3 (leu2⁻, ade1⁻) by mixing the strains on a YEHD complete media plate. To select for diploids, the mated strains were replica plated onto leu⁻ minimal medium containing 2% sucrose as the sole carbon source. After isolating single colonies, the diploids were sporulated, and asci were dissected by standard techniques. The KHY-107 strain was isolated as a single spore and characterized as cir⁺, ade1⁺, leu2⁻, and mnn9- (by Schiff stain technique).

KHY107 (cir 0) was derived from strain KHY107 (cir⁺) as described by Broach [Methods in Enzymology, 101, Part C, pp 307-325, (1983)]. The cured strain was made ura3⁻ by integrating a disrupted ura3 gene. The resulting strain, KHY-107ura3Δ, was grown in rich media to allow the accumulation of spontaneous mutations and a canavanine resistant mutant was selected. The mutant strain, CF55 (Mata leu2-2,112 ura3Δ can1 cir° mnn9-), was shown by complementation tests to be can1⁻. The GAL10pGAL4 expression cassette was integrated into the HIS3 gene of CF55 (Methods in Enzymology, 1990, 185 pp297-309) to yield the final host strain CF52 (Mata leu2-2,112 ura3Δ can1 his3Δ::GAL10pGAL4-URA3, cir° mnn9⁻).

### EXAMPLE 6

Yeast Transformation and Seed Establishment for HBsAg in
CF52

The bidirectinal vector described in Example 4 was used to transform S. cerevisiae strain CF52 (Example 5). Clones were selected on minimal medium (leu-, containing 1M sorbitol), established as frozen stocks (in 17% glycerol) and evaluated as described below.

### EXAMPLE 7

Growth and Expression of S Particles in Yeast CF52
(mnn9-)

Clones of yeast containing the expression plasmid described in Example 6 above were plated onto leu⁻ selective agar plates containing 1M sorbitol and incubated at 30°C for 2-3 days. These yeast were inoculated into 5-7 mL of complex YEHDS (YEHD + 0.1M sorbitol) and the cultures were incubated at 30°C with aeration for 12-18 hours. Flasks containing 50 mL YEHDS + 2% galactose media were inoculated from the above culture (to an initial A₆₀₀ = 0.1) and incubated at 30°C with shaking (350 rpm) for 72 hours to a final A₆₀₀ of 10-16. Samples of 10 A₆₀₀ units were dispensed into tubes, and the yeast cells were pelleted at 2,000 x g for 10 minutes. The pellets either were assayed directly or stored at -70°C for future assay. At the time of assay, the pellets were resuspended in 0.3 mL of phosphate-buffered saline containing 2mM PMSF. Yeast cells were broken by: 1) the addition of 200-300 mg of washed glass beads (0.45 mm), 2) agitation on a vortex mixer for 15 minutes, 3) addition of TRITON X-100 to 0.5% (v/v), 4) agitation on a vortex mixer for 2 minutes, and 5) incubation at 4°C for 10-15 minutes. Cellular debris and glass beads were removed by centrifugation at 13,000 x g for 10 minutes. The clarified supernatant fluid was removed and analyzed for protein [by the method of Lowry et al., J. Biol. Chem., 193, 265 (1951)] and for HBsAg by (AUSRIA^{R}) assay (Abbott).

### EXAMPLE 8

Large Scale Growth of S. cerevisiae Producing HBsAg
mixed particles in Fermentors

The frozen recombinant yeast culture was inoculated onto leu⁻ plates containing 1M sorbitol. The plates were incubated inverted at 28°C for 2-3 days. The growth on the plates was resuspended in YEHDS and the resuspended growth was transferred into 2-L Erlenmeyer flask containing 500 mL of YEHDS, and 2% galactose. The flask was incubated at 28°C and 350 rpm in a controlled environment shaker incubator for 18-22 hours. These seed cultures then were used to inoculate the production stage vessels.

An inoculum (1-5% v/v) from one or more flasks was transferred into 16-L or 250-L fermentors containing 10-L or 200-L of YEHDS, respectively. The 16-L fermentors were operated at 500 rpm, 5 L/min air, and 28°C. The 250-L fermentors were operated at 160 RPM, 60 L/min air and 28°C. The fermentors were harvested 40-46 hrs after inoculation with the seed culture. Optical density values of 15.0 A₆₆₀ units typically were obtained. Harvesting consisted of concentrating the cells using a hollow fiber filtering device followed by washing the cells in buffered salt solutions. Cell slurries were assayed as described below or stored frozen at -70°C for further processing and analysis.

Small samples (0.6 mL) of 20% washed cell slurries were broken using glass beads (0.45-0.52 mm) in 1.5-mL Eppendorf tubes. PMSF (6.5 µl of 200 mM stock) was added as a protease inhibitor. Aliquots were removed from the tubes after breakage and frozen at -70°C for immunoblot analysis. TRITON X-100 was added to the remaining sample in the tubes to a final concentration of 0.5%, and the samples were mixed briefly and incubated at 4°C for 20-40 min. The cell debris was removed by centrifugation and the clarified cell extract assayed for S antigen by (by Ausria^{R}), and protein (by Lowry).

### EXAMPLE 9

### Purification of Mixed particles in particulate form by means of immune affinity chromatography

Recombinant S. cerevisiae, constructed as described in Example 6, were grown in either flasks or fermentors. The yeast cells were harvested by microfiltration in an Amicon DC-10 unit, suspended in 30 ml buffer A [0.1M Na₂HPO₄, pH 7.2, 0.5M NaCl], and broken in a Stansted pressure cell for seven passages at 75-85 pounds per square inch. The broken cell suspension (31 gm wet cell weight) was diluted with 120 ml buffer A, TRITON X-100 was added to a final concentration of 0.5% (w/v), and the suspension was clarified by centrifugation at 10,000 x g for 20 min. at 4°C. The clarified broth was decanted and incubated with Sepharose 4B coupled with antibodies to HBsAg [McAleer et al., Nature 307: 178 (1984)] for about 19 hours at 4°C to adsorb the antigen onto the resin. After the incubation period, the slurry was warmed to room temperature for all subsequent steps and degassed under vacuum for 15 min. The degassed slurry was poured into a 2.5 x 40 cm column. When the column had been packed fully, unbound protein was washed away with buffer A. The antigen was eluted with 3M KSCN in buffer A. Fractions containing antigen were dialyzed against 0.007M Na₂HPO₄, pH 7.2, 0.15M NaCl at 4°C and pooled to form the Dialyzed Affinity Pool containing 1.08 mg of protein in 20 ml. Sixteen ml of Dialyzed Affinity Pool was diluted to 40 mcg/ml with 5.6 ml 0.006M Na₂HPO₄, pH 7.2, 0.15M NaCl. The product was sterilized by filtration through a Millex-GV 0.22 µ membrane. The identity of the product in the Dialyzed Affinity Pool was verified by the detection of HBsAg by Ausria^{R} reactivity and of a band of of about 24kD by immunoblott anti-24(S) sera.

### EXAMPLE 10

### Large Scale Purification of Recombinant HBsAg

About 250g of frozen cell paste (producing particles of recombinant S antigens) was resuspended to 17% wet weight/volume (about 1500 ml) in phosphate buffered saline solution (PBS). The cells were heated to 45°C by immersion in a water bath. The cells were held at 45°C for 15 minutes and then cooled on ice to about 10°C. The cells were then disrupted by two passages through a Gaulin homogenizer.

Following homogenization, 10% TRITON X-100 was added to a final concentration of 0.3% and mixed for about 15 minutes. The cell extract was then centrifuged at 3,600 x g for 20 minutes at 4°C, and the supernatant was collected.

The superantant was then passaged over a column containing about 200g of XAD-2 resin to remove the TRITON X-100. The effluent was then passaged directly over a column containing about 150g of wide pore silica with a pore size of about 1,500 angstrom and a particle size of about 50 microns. The columns used were 5 cm diameter (Pharmacia) and were run at a flow rate of about 200 ml per hour.

The silica column was washed with PBS until the A₂₈₀ returned to baseline.

The antigen was eluted from the silica column using first, cold borate buffer (50 mM, pH 8.7, 4°C) at a flow rate of about 500 ml per hour, until a rise in the A₂₈₀ was observed. Once the A₂₈₀ began to rise the column was heated to 55°C and 55°C borate buffer was run through the column at about 500 ml per hour. The eluate containing mixed particle antigen (about 1L) was collected on ice. The eluate was then concentrated to about 200 ml by difiltration against 50 mM borate buffer at pH 8.7, using a hollow fiber diafiltration unit with a molecular weight cutoff of 10⁵. The S protein was then filtered through a 0.2 micron filter and stored. The product was found to be stable with no significant degradation observed on Immunoblot analysis.

### EXAMPLE 11

### Assay of Carbohydrate Content of the Recombinant HBV Surface Proteins

The carbohydrate content of the recombinant HBV surface proteins is determined by the method of Dubois, M. et al., Anal. Chem., 28, pp.350, 1956. The general principle of this procedure is that simple sugars, oligosaccharides, polysaccharides and their derivatives, including the methyl ethers with free or potentially free reducing groups, give an orange yellow color when treated with phenol and concentrated sulfuric acid. The amount of color produced at a constant phenol concentration is proportional to the amount of sugar present.

To determine the carbohydrate content of HBsAg produced in wild-type yeast strain and produced in the mnn9⁻ yeast strain, 1 mL of a solution containing between 10 to 70 µg of protein is placed in a test tube. A series of carbohydrate standards and blank samples are prepared. Phenol solution is added to each tube, the tubes are mixed, and 5 mL of a 96% sulfuric acid solution is added and mixed. The tubes are incubated at room temperature for 10 minutes, mixed, and incubated at 25 to 30°C for 20 minutes. The samples are read in spectrophotometer (A₄₉₀ for hexoses and methylated hexoses, and A₄₈₀ for pentoses, uronic acid, and their methylated derivatives) and the amount of carbohydrate in the HBV surface protein samples is determined by comparison with the carbohydrate standards.

Based on these results, a ratio of the amount of carbohydrate to protein present in each sample is calculated by dividing the micrograms of carbohydrate by the micrograms of protein in the sample.

## Claims

1. A multivalent vaccine comprising individual HBsAg particles comprising multiple serotypes and/or mutants of S protein of, free from other hepatitis B virus proteins.

2. The vaccine according to Claim 1 wherein the hepatitis B virus S proteins are of the serotypes adw, ayw, adr, ayr, or adyw.

3. The vaccine according to Claim 2 wherein the hepatitis B virus S proteins are of the serotypes adw and ayw.

4. The vaccine according to Claim 3 wherein the multiple serotypes of S protein contain substantially reduced entrapped carbohydrate content.

5. The vaccine according to Claim 4 wherein the hepatitis B virus S proteins are produced in a recombinant host cell.

6. The vaccine according to Claim 5 wherein the recombinant host cell is yeast.

7. The vaccine according to Claim 6 wherein the yeast host is Saccharomyces cerevisiae.

8. Individual HBsAg particles comprising multiple serotypes of S protein, free from other HBV proteins.

9. The HBsAg particles of Claim 8, wherein the S protein serotypes are selected from the group adw, ayw, adr, ayr, or adyw.

10. The HBsAg particles of Claim 9, wherein the S protein serotypes are adw and ayw.

11. The HBsAg particles of Claim 8, wherein the particles contain substantially reduced entrapped carbohydrate content.

12. The HBsAg particles of Claim 11, wherein the S protein serotypes are selected from group adw, ayw, adr, ayr, adyw.

13. The HBsAg particles of Claim 12, wherein the S protein serotypes are adw and ayw.

14. The HBsAg particles of Claim 8, wherein the particles are produced in a recombinant host.

15. The HBsAg particles of Claim 14, wherein the recombinant host is yeast.

16. The HBsAg particles of Claim 15, wherein the yeast host is Saccharomyces cerevisiae.

17. The HBsAg particles of Claim 11, wherein the particles are produced in a recombinant host.

18. The HBsAg particles of Claim 17, wherein the recombinant host is yeast.

19. The HBsAg particles of Claim 18, wherein the yeast host is Saccharomyces cerevisiae.

20. The HBsAg particles of Claim 18, wherein the yeast host is mnn9-.

21. The HbsAg particles of Claim 8 for use as a diagnostic reagent capable of detecting diseases caused by multiple HBV serotypes, mutant hepatitis B viruses and hepatitis B- related viruses.


(1) GENERAL INFORMATION:

(i) APPLICANT: Kniskern, P. J.
Hagopian, A.
Burke, P.

(ii) TITLE OF INVENTION: Multiple Hepatitis B Virus Surface
Proteins Which Form Particles With Reduced Host
Carbohydrate Content

(iii) NUMBER OF SEQUENCES: 11

(iv) CORRESPONDENCE ADDRESS:
(A) ADDRESSEE: Merck & Co., Inc.
(B) STREET: P.O. Box 2000
(C) CITY: Rahway
(D) STATE: New Jersey
(E) COUNTRY: US
(F) ZIP: 07065-0900

(v) COMPUTER READABLE FORM:
(A) MEDIUM TYPE: Floppy disk
(B) COMPUTER: IBM PC compatible
(C) OPERATING SYSTEM: PC-DOS/MS-DOS
(D) SOFTWARE: PatentIn Release #1.0, Version #1.25

(vi) CURRENT APPLICATION DATA:
(A) APPLICATION NUMBER: Not Available
(B) FILING DATE: Not Available
(C) CLASSIFICATION: Not Available

(viii) ATTORNEY/AGENT INFORMATION:
(A) NAME: Pfeiffer, Hesna J.
(B) REGISTRATION NUMBER: 22,640
(C) REFERENCE/DOCKET NUMBER: 18345

(ix) TELECOMMUNICATION INFORMATION:
(A) TELEPHONE: (908) 594-4251
(B) TELEFAX: (908) 594-4720

(2) INFORMATION FOR SEQ ID NO:1:

(i) SEQUENCE CHARACTERISTICS:
(A) LENGTH: 10 base pairs
(B) TYPE: nucleic acid
(C) STRANDEDNESS: single
(D) TOPOLOGY: linear

(ii) MOLECULE TYPE: DNA (genomic)

(xi) SEQUENCE DESCRIPTION: SEQ ID NO:1:

ACAAAACAAA 10

(2) INFORMATION FOR SEQ ID NO:2:

(i) SEQUENCE CHARACTERISTICS:
(A) LENGTH: 30 base pairs
(B) TYPE: nucleic acid
(C) STRANDEDNESS: single
(D) TOPOLOGY: linear

(ii) MOLECULE TYPE: DNA (genomic)

(xi) SEQUENCE DESCRIPTION: SEQ ID NO:2:

AATTCAAGCT TACAAAACAA AATGCAGTGG 30

(2) INFORMATION FOR SEQ ID NO:3:

(i) SEQUENCE CHARACTERISTICS:
(A) LENGTH: 30 base pairs
(B) TYPE: nucleic acid
(C) STRANDEDNESS: single
(D) TOPOLOGY: linear

(ii) MOLECULE TYPE: DNA (genomic)

(xi) SEQUENCE DESCRIPTION: SEQ ID NO:3:

GTTCGAATGT TTTGTTTTAC GTCACCTTAA 30

(2) INFORMATION FOR SEQ ID NO:4:

(i) SEQUENCE CHARACTERISTICS:
(A) LENGTH: 16 base pairs
(B) TYPE: nucleic acid
(C) STRANDEDNESS: single
(D) TOPOLOGY: linear

(ii) MOLECULE TYPE: DNA (genomic)

(xi) SEQUENCE DESCRIPTION: SEQ ID NO:4:

ATACATTTAA AGCTTG 16

(2) INFORMATION FOR SEQ ID NO:5:

(i) SEQUENCE CHARACTERISTICS:
(A) LENGTH: 18 base pairs
(B) TYPE: nucleic acid
(C) STRANDEDNESS: single
(D) TOPOLOGY: linear

(ii) MOLECULE TYPE: DNA (genomic)

(xi) SEQUENCE DESCRIPTION: SEQ ID NO:5:

TGTAAATTTC GAACCTAG 18

(2) INFORMATION FOR SEQ ID NO:6:

(i) SEQUENCE CHARACTERISTICS:
(A) LENGTH: 45 base pairs
(B) TYPE: nucleic acid
(C) STRANDEDNESS: single
(D) TOPOLOGY: linear

(ii) MOLECULE TYPE: DNA (genomic)

(xi) SEQUENCE DESCRIPTION: SEQ ID NO:6:

AATTCAAGCT TACAAAACAA AATGGAGAAC ATCACATCAG GATTC 45

(2) INFORMATION FOR SEQ ID NO:7:

(i) SEQUENCE CHARACTERISTICS:
(A) LENGTH: 45 base pairs
(B) TYPE: nucleic acid
(C) STRANDEDNESS: single
(D) TOPOLOGY: linear

(ii) MOLECULE TYPE: DNA (genomic)

(xi) SEQUENCE DESCRIPTION: SEQ ID NO:7:

GTTCGAATGT TTTGTTTTAC CTCTTGTAGT GTAGTCCTAA GGATC 45

(2) INFORMATION FOR SEQ ID NO:8:

(i) SEQUENCE CHARACTERISTICS:

(A) LENGTH: 80 base pairs

(B) TYPE: nucleic acid

(C) STRANDEDNESS: single

(D) TOPOLOGY: linear

(ii) MOLECULE TYPE: DNA (genomic)

(xi) SEQUENCE DESCRIPTION: SEQ ID NO:8:

AGCTTACAAA ACAAAATGGA GAACATCACA TCAGGATTCC TAGGGCCCCT TCTCGTGTTA 60

CAGGCCGGCT TTTTCTTGTT 80

(2) INFORMATION FOR SEQ ID NO:9:

(i) SEQUENCE CHARACTERISTICS:

(A) LENGTH: 76 base pairs

(B) TYPE: nucleic acid

(C) STRANDEDNESS: single

(D) TOPOLOGY: linear

(ii) MOLECULE TYPE: DNA (genomic)

(xi) SEQUENCE DESCRIPTION: SEQ ID NO:9:

AACAAGAAAA AGCCGGCCTG TAACACGAGA AGGGGCCCTA GGAATCCTGA TGTGATGTTC 60

TCCATTTTGT TTTGTA 76

(2) INFORMATION FOR SEQ ID NO:10:

(i) SEQUENCE CHARACTERISTICS:
(A) LENGTH: 29 base pairs
(B) TYPE: nucleic acid
(C) STRANDEDNESS: single
(D) TOPOLOGY: linear

(ii) MOLECULE TYPE: DNA (genomic)

(xi) SEQUENCE DESCRIPTION: SEQ ID NO:10:

AATTGTCGAC AGCTAGCTGA ATTCCCGGG 29

(2) INFORMATION FOR SEQ ID NO:11:

(i) SEQUENCE CHARACTERISTICS:
(A) LENGTH: 29 base pairs
(B) TYPE: nucleic acid
(C) STRANDEDNESS: single
(D) TOPOLOGY: linear

(ii) MOLECULE TYPE: DNA (genomic)

(xi) SEQUENCE DESCRIPTION: SEQ ID NO:11:

AGCTCCCGGG AATTCAGCTA GCTGTCGAC 29